# EUROPÄISCHE PATENTSCHRIFT

(11) **EP 0 629 607 B1**
(45) Veröffentlichungstag und Bekanntmachung des Hinweises auf die Patenterteilung: **31.10.2001**
(21) Anmeldenummer: 94108570.6
(22) Anmeldetag: 03.06.1994
(51) Int. Cl.: C07C 229/30, C08G 18/18, C08G 18/67

(54) **Tertiäre Aminogruppen aufweisende Verbindungen, ein Verfahren zu ihrer Herstellung und ihre Verwendung als Katalysatoren**
Compounds containing tertiary amino groups, process for their preparation and use as catalyst
Composés contenant un groupement amino tertiaire, procédé pour leur préparation et leur utilisation comme catalysateur

(30) Priorität: 16.06.1993 DE 4319948
(43) Veröffentlichungstag der Anmeldung: 21.12.1994
(73) Patentinhaber: BAYER AG, 51368 Leverkusen (DE)
(72) Erfinder: Sanders, Josef, Dr., D-51373 Leverkusen (DE); Liman, Ulrich, Dr., D-40764 Langenfeld (DE); König, Klaus, Dr., D-51519 Odenthal (DE)

(56) Entgegenhaltungen:
- EP-A- 0 429 169
- EP-A- 0 469 545
- EP-A- 0 477 697
- EP-A- 0 502 516
- EP-A- 0 550 065
- DE-A- 1 935 484
- DE-A- 2 414 456
- FR-A- 2 305 176
- US-A- 4 101 462
- EUROPEAN POLYMER JOURNAL, Bd. 13, Nr. 6, 1977, OXFORD GB, Seiten 531-536, XP000196217 DE COINTET P. ET AL.: "Stabilisants thermiques du polychlorure de vinyle: Dérivés de l'acide Bêta-Aminocrotonique"

## Beschreibung

Die Erfindung betrifft neue, tertiäre Aminogruppen aufweisende Aminocrotonsäureester, ein Verfahren zu ihrer Herstellung sowie ihre Verwendung als Katalysatoren bzw. Aktivatoren bei der Herstellung von Polyurethanschaumstoffen.

Die Herstellung von Aminocrotonsäureestern durch Umsetzung von Acetessigsäureestem ein- oder mehrwertiger Alkohole, insbesondere höhermolekularer Polyetherpolyole mit Ammoniak, Aminen oder Aminoalkoholen ist bereits aus zahlreichen Vorveröffentlichungen bekannt. So beschreibt die DE-A-1 935 484 die Umsetzung der Acetessigsäureester mit Ammoniak oder Monoaminen. Die DE-A-1 935 485 bzw. die EP-A 0 429 169 ihrerseits beschreiben die Herstellung von Aminocrotonsäureestern durch Umsetzung der genannten Acetessigsäureester mit Diaminen oder Aminoalkoholen. Bei den Verfahrensprodukten der genannten Vorveröffentlichungen handelt es sich um modifizierte Polyole, die endständig alkoholische Hydroxylgruppen bzw. primäre oder sekundäre Aminogruppen aufweisen und die daher interessante Reaktionspartner für organische Polyisocyanate bei der Herstellung von Polyurethanen bzw. Polyharnstoffen nach dem Isocyanat-Polyadditionsverfahren darstellen.

EP-A 469 545 beschreibt sekundäre Hydroxylgruppen aufweisende Amine, die als nicht migrierende Katalysatoren für Polyurethane vorgeschlagen werden. US-PS 4 101 462 offenbart ein Verfahren zur Herstellung von Polyurethanen, bei dem ein Reaktionsprodukt eines N,N-disubstituierten Aminoalkylenamins mit einem Alkylenoxid als Katalysator Verwendung findet. Auch diese Verbindungen weisen primäre oder sekundäre Hydroxylgruppen auf.

Wie jetzt überraschend gefunden wurde, stellen Aminocrotonsäureester der nachstehend näher beschriebenen Art mit endständig angeordneten tert.-Aminogruppen besonders wertvolle, bei gleichzeitigem Vorliegen von Hydroxylgruppen einbaufähige Katalysatoren für das Isocyanat-Polyadditionsverfahren dar.

Besonders vorteilhaft können die nachstehend näher beschriebenen erfindungsgemäßen Verbindungen als Katalysatoren zur Herstellung von Verbundkörpern durch Hinterschäumen von Kunststoffolien eingesetzt werden, die in größerem Umfang z.B. zur Herstellung von Polstermöbeln verwendet werden. Hierbei hat es sich oft gezeigt, daß Katalysatoren, z.B. niedermolekulare tertiäre Amine wie Triethylendiamin (Dabco) oder Triethylamin, in die Deckmaterialien diffundieren und diese, gegebenenfalls unter dem Einfluß von Sonnenlicht und Wärme, schädigen. Zudem gelangen diese Stoffe auch in die Umgebung und tragen zum sogenannten Fogging bei, nicht selten unter Geruchsbelästigung. Die Verwendung von organischen Zinnverbindungen führt zwar im allgemeinen zu einer Verbesserung des Alterungsverhaltens, jedoch wegen der Hydrolyseanfälligkeit der organischen Zinnverbindungen im wasserhaltigen Polyol zu Problemen bezüglich der konstanten Aktivierung der Polyolkomponente über einen längeren Zeitraum.

Wie überraschend gefunden wurde, können die angesprochenen Probleme unter Verwendung der nachstehend näher beschriebenen erfindungsgemäßen Verbindungen als Katalysatoren in optimaler Weise gelöst werden.

Gegenstand der Erfindung sind tertiäre Aminogruppen aufweisende Verbindungen der Formel (I) 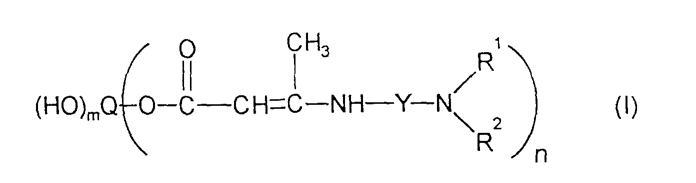 in welcher
- Q: für den Rest steht, der durch Entfernung der Hydroxylgruppe(n) aus einem von 1,4-Butandiol verschiedenen 2-6-wertigen Alkohol des Molekulargewichts 62 bis 4000 erhalten wird,
- Y: für einen zweiwertigen aliphatischen Kohlenwasserstoffrest mit 2 bis 6 Kohlenstoffatomen steht, wobei zwischen den beiden Stickstoffatomen mindestens zwei Kohlenstoffatome angeordnet sind,
- R¹ und R²: für gleiche oder verschiedene Reste stehen und Alkylreste bedeuten, die zusammen mit dem Stickstoffatom und gegebenenfalls weiteren, gegebenenfalls alkylsubstituierten Heteroatomen auch zu einem gesättigten heterocyclischen Ring verknüpft sein können,
- m: eine Zahl von 0 bis 5 und
- n: eine Zahl von 1 bis 6 bedeuten, mit der Maßgabe, daß die Summe m + n eine Zahl von 2 bis 6 ergibt.

Gegenstand der Erfrindung ist auch ein Verfahren zur Herstellung dieser Verbindungen, welches dadurch gekennzeichnet ist, daß man Verbindungen der Formel (II) 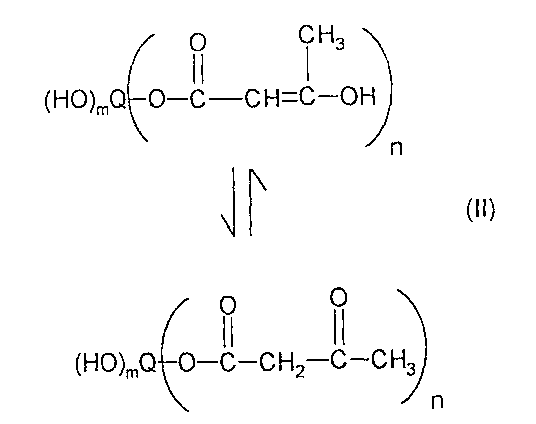 mit Verbindungen der Formel (III) 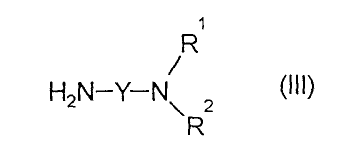 im Sinne einer Kondensationsreaktion zu Reaktionen bringt, wobei
- Q, Y, R¹, R², m und n: die soeben genannte Bedeutung haben.

Gegenstand der Erfindung ist auch die Verwendung der erfindungsgemäßen Verbindungen als gegebenenfalls einbaufähige Katalysatoren für die Isocyanat-Additionsreaktion bei der Herstellung von Polyurethanschaumstoffen nach dem Isocyanat-Polyadditionsverfahren, insbesondere von Verbundkörpern durch Hinterschäumen von Kunststoffolien mit einem zu Polyurethanschaumstoff ausreagierenden Reaktionsgemisch.

Die Variablen Q, Y, R¹, R², m und n haben vor- und nachstehend die bereits genannte Bedeutung. Die Bedeutung dieser Variablen ist wie folgt:
- Q: steht für den Rest, wie er durch Entfernung der Hydroxylgruppen aus einem 2- bis 6-wertigen Alkohol des Molekulargewichtsbereichs 62 bis 4000 erhalten wird, insbesondere für den Rest, wie er durch die Entfernung der Hydroxylgruppen aus einem di- bis hexafunktionellen Polyetherpolyol des Molekulargewichtsbereichs 200 bis 2000, insbesondere 250 bis 1000 erhalten wird.
- Y: steht bevorzugt für einen Ethylen-, 1,2-Propylen- oder Trimethylenrest.
- R¹ und R²: stehen vorzugsweise für gleiche C₁-C₃-Alkylreste oder sie bilden zusammen mit dem Stickstoffatom und gegebenenfalls einem Sauerstoffatom oder einem weiteren, C₁-C₃-Alkyl-substituierten Stickstoffatom einen gesättigten heterocyclischen Ring mit 6 Ringgliedern.
- m: steht für eine ganze oder (im statistischen Mittel) oder gebrochene Zahl von 0 bis 5 und
- n: für eine ganze oder (im statistischen Mittel) oder gebrochene Zahl von 1 bis 6, wobei die Summe m + n einen Wert von 2 bis 6 ergibt.

Besonders bevorzugte erfindungsgemäße Verbindungen der Formel (I) sind solche, die pro Molekül im statistischen Mittel weniger als 0,2, insbesondere 0 oder mehr als 1,8, insbesondere mindestens 2 Hydroxylgruppen aufweisen, so daß sie bei ihrer Verwendung als Katalysatoren bei der Isocyanat-Additionsreaktion nicht als Kettenabbrecher wirken.

Zur Herstellung der in der Keto- und Enolform vorliegenden Acetessigsäureester der Formel (II) können Alkohole der Formel (IV)

Q(OH)ₘ₊ₙ (IV)

mit Acetessigssäurealkylester im Sinne einer Umesterung bei Temperaturen von 60 bis 210°C, bevorzugt 80 bis 160°C, unter destillativer Entfernung der Alkoholkomponente des eingesetzten Acetessigsäurealkylesters umgesetzt werden. Hierbei können die Acetessigsäurealkylester im Unter- oder Überschuß, bezogen auf die vorhandenen Hydroxyl-Äquivalente, eingesetzt werden. So ist zur Erzielung eines möglichst vollständigen Umsatzes der Hydroxylgruppen ein Überschuß an Acetessigsäurealkylester zweckmäßig bzw. erforderlich, während bei Einsatz eines Unterschusses an Acetessigsäurealkylester gezielt Verbindungen erhalten werden, die noch freie Hydroxylgruppen aufweisen und daher im Sinne der Urethanchemie als einbaufähig gelten. Die Reaktion wird bevorzugt ohne Lösungsmittel durchgeführt, jedoch kann die Verwendung von Lösungsmitteln in einigen Fällen zweckmäßig bzw. erforderlich sein, z.B. bei Einsatz schwerlöslicher Hydroxylverbindungen. In solchen Fällen kommen Lösungsmittel in Betracht, die nicht mit den Reaktionskomponenten reagieren und einen zur destillativen Trennung von der Alkoholkomponente des eingesetzten Acetessigsäurealkylesters ausreichend hohen Siedepunkt aufweisen. Geeignet sind beispielsweise Kohlenwasserstoffe wie Toluol oder Xylol, halogenierte Kohlenwasserstoffe wie Chlorbenzol oder Dichlorbenzol, Ether wie Ethylenglykoldimethylether, Diethylenglykoldimethylether, Diphenylether oder Dioxan und Amide wie Dimethylformamid, Dimethylacetamid oder N-Methylpyrrolidon. In manchen Fällen kann der Zusatz von Veresterungskatalysatoren wie Dibutyl-zinnoxid, Zinndichlorid oder Tetrabutyltitanat vorteilhaft sein, jedoch ist eine Arbeitsweise ohne Katalysatorzusatz bevorzugt.

Im allgemeinen werden die Reaktionskomponenten solange unter den beschriebenen Reaktionsbedingungen umgesetzt, bis die Destillation der Alkoholkomponente des eingesetzten Acetessigsäurealkylesters beendet ist, bzw. bis die theoretisch zu erwartende OH-Zahl erreicht ist. Hierzu ist, besonders gegen Ende der Reaktion, das Anlegen von Vakuum vorteilhaft bzw. erforderlich.

Geeignete Alkohole der Formel (IV) sind beispielsweise 2-bis 6-wertige niedermolekulare Alkohole des Molekulargewichtsbereichs 62 bis 399 wie z.B. Ethylenglykol, Propylenglykol, die isomeren Butandiole mit Ausnahme von 1,4-Butandiol, Hexandiole oder Octandiole, Glycerin, Trimethylolpropan, Pentaerythrit, Sorbit, Saccharose oder niedermolekulare Alkoxylierungsprodukte solcher Alkohole des genannten Molekulargewichtsbereichs.

Weiterhin geeignete Alkohole sind beispielsweise die aus der Polyurethanchemie an sich bekannten 2 bis 6 Hydroxylgruppen aufweisenden Polyacetale, Polythioether, Polycarbonate, Polyamide, Polysiloxane, Polyester, Polylactone und insbesondere Polyether des Molekulargewichtsbereichs 400 bis 4000 und insbesondere 400 bis 3000. Ganz besonders bevorzugt handelt es sich bei den Alkoholen der Formel (IV) um (m+n)-wertige Polyetherpolyole des Molekulargewichtsbereichs 200 bis 2000, insbesondere 250 bis 1000. Alle hier, sowie vor- und nachstehend gemachten Ausführungen bezüglich des Molekulargewichts der Polyole der Formel (IV) beziehen sich auf das aus Hydroxylfunktionalität und Hydroxylgruppengehalt errechenbare Molekulargewicht.

Beliebige Gemische der beispielhaft genannten mehrwertigen Alkohole der Formel (IV) können ebenfalls zur Herstellung der als erfindungsgemäßes Ausgangsmaterial eingesetzten Acetessigsäureester verwendet werden. Die genannten Molekulargewichte beziehen sich auf das aus Hydroxylfunktionalität und Hydroxylgruppengehalt berechenbare (mittlere) Molekulargewicht.

Als Acetessigsäurealkylester, die mit den Alkoholen der Formel (IV) im Sinne einer Umesterungsreaktion umgesetzt werden sind beliebige C₁-C₆-Alkylester der Acetessigssäure wie beispielsweise Methyl-, Ethyl-, n-Propyl-, n-Butyl-, tert.-Butyl- oder n-Hexyl-acetoacetat geeignet. Besonders bevorzugt werden Methyl-, Ethyl- oder tert.- Butylacetoacetat eingesetzt.

Als primär/tertiäre Diamine der Formel (III) kommen beim erfindungsgemäßen Verfahren solche zum Einsatz, für welche Y, R¹ und R² die obengenannte Bedeutung bzw. bevorzugte Bedeutung haben. Beispielhaft genannt seien 1-(Dimethylamino)-3-aminopropan, 1-(Diethylamino)-3-aminopropan, 1-(Di-n-propylamino)-3-aminopropan, 1-(Dimethylamino)-2-methyl-3-amino-propan, 1-(Dimethylamino)-4-aminobutan oder -5-aminopentan, N-(2-Aminoethyl)-morpholin, N-(3-Aminopropyl)-morpholin, N-(2-Aminoethyl)-piperidin, N-(3-Aminopropyl)-piperidin oder N(3-Aminopropyl)-N'-n-propyl-piperazin. Prinzipiell ist auch die Verwendung von Gemischen derartiger Verbindungen möglich. Bevorzugt wird 1-(Dimethylamino)-3-aminopropan als primäre/tertiäres Diamin der Formel (III) verwendet.

Die beispielhaft genannten Verbindungen der Formel (III) sind bekannt und zum Teil kommerziell erhältlich.

Zur Durchführung des erfindungsgemäßen Verfahrens werden die Acetessigsäureester der Formel (II) mit den primär/tertiären Diaminen der Formel (III) bei Temperaturen von 10 bis 150, vorzugsweise 20 bis 120°C, vorzugsweise unter anschließender und/oder gleichzeitiger destillativer Entfernung des Reaktionswassers zu den erfindungsgemäßen Aminocrotonsäureestern der Formel (I) kondensiert. Hierbei wird die Menge der eingesetzten Amine der Formel (III) vorzugsweise so bemessen, daß pro Äquivalent Acetoacetat 0,8 bis 1,0 Mol an primären Aminogruppen zur Verfügung steht. In Ausnahmefällen, beispielsweise wenn an einer möglichst niedrigen Viskosität der Umsetzungsprodukte Interesse besteht, können die Diamine der Formel (III) auch in einer unter 0,8 Mol Aminogruppen pro Mol Acetoacetatgruppen liegenden Menge zum Einsatz gelangen. Die Verwendung eines Aminüberschusses ist dagegen weniger bevorzugt, da nur äquimolare Mengen Amin verbraucht werden, so daß die überschüssige Menge an Aminkomponente in den Endprodukten in freier Form vorliegt und entweder im Produkt belassen oder daraus, z.B. durch Destillation, entfernt werden muß. Die Reaktion wird bevorzugt ohne Lösungsmittel durchgeführt, jedoch kann die Verwendung von Lösungsmitteln in einigen Fällen zweckmäßig bzw. erforderlich sein, z.B. wenn die Reaktionspartner keine ausreichende Verträglichkeit aufweisen, oder wenn die Viskosität der Reaktionsmischung zu hoch liegt. In solchen Fällen kommt die Verwendung von Lösungsmitteln in Betracht, die nicht mit den Reaktionskomponenten reagieren und nach beendeter Reaktion wieder möglichst leicht, z.B. durch Destillation aus dem Reaktionsgemisch entfernt werden können. Besonders geeignet sind solche Lösungsmittel, die ein Azeotrop mit Wasser bilden und somit die Auskreisung des gebildeten Reaktionswassers, z.B. durch Kochen am Wasserabscheider, erleichtern. Beispiele geeigneter Lösungsmittel sind Kohlenwasserstoffe wie Toluol oder Xylol, halogenierte Kohlenwasserstoffe wie Methylenchlorid, Chlorbenzol oder Dichlorbenzol, Ether wie Ethylenglykoldimethylether, Diethylenglykoldimethylether, Diphenylether, Tetrahydrofuran oder Dioxan, Amide wie Dimethylformamid, Dimethylacetamid oder N-Methylpyrrolidon.

Im allgemeinen werden die Reaktionskomponenten solange unter den beschriebenen Reaktionsbedingungen umgesetzt, bis die theoretisch zu erwartende Menge Wasser abdestilliert ist, oder bis das Verschwinden der Acetoacetat-Carboxylbande bei 1740 cm⁻¹ im IR-Spektrum der Reaktionsmischung vollständigen Umsatz der Acetoacetat-Gruppen anzeigt. Zur Entfernung letzter Wasserspuren ist, besonders gegen Ende der Reaktion, das Anlegen von Vakuum vorteilhaft bzw. erforderlich.

Falls die Reaktionspartner bei der Durchführung des erfindungsgemäßen Verfahrens in äquivalenten Mengen (1 Mol primäre Aminogruppen pro Äquivalent Acetoacetat) zum Einsatz gelangen, entstehen im wesentlich von Nebenprodukten freie erfindungsgemäße tertiäre Amine der Formel (I). Im Falle der Verwendung eines Überschusses an Acetessigsäureester der Formel (II) fallen die erfindungsgemäßen Verbindungen selbstverständlich in Form von Gemischen mit untergeordneten Mengen an Nebenprodukten an. Bei diesen Nebenprodukten handelt es sich um unumgesetzte Ausgangsmaterialien der Formeln (II) und/oder um Umsetzungsprodukte, in denen sowohl nicht umgesetzte Acetylessigsäuregruppen als auch Struktureinheiten der Formel (V) 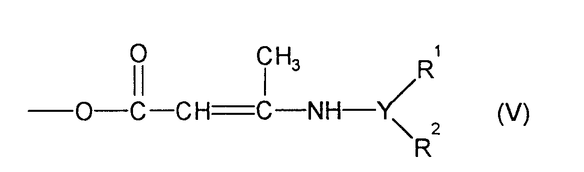 vorliegen. Jedoch auch im Falle dieser Gemische stellen die erfindungsgemäßen tertiäre Aminogruppen aufweisenden Verbindungen der Formel (I) stets die Hauptkomponente dar. Die Verwendbarkeit der Gemische im Sinne der erfindungsgemäßen Verwendung wird durch das Vorliegen der genannten Nebenprodukte nicht nennenswert beeinträchtigt.

Die erfindungsgemäßen, tertiäre Aminogruppen aufweisenden Verbindungen der Formel (I) bzw. ihre Gemische mit den genannten Nebenprodukten sind weitgehend geruchsfrei. Durch geeignete Wahl der Ausgangsmaterialien, insbesondere der den erfindungsgemäßen Verbindungen zugrundeliegenden Alkohole der Formel (IV) beim erfindungsgemäßen Verfahren werden als erfindungsgemäße Verfahrensprodukte Verbindungen erhalten, die eine ausgezeichnete Verträglichkeit mit den in der Polyurethanchemie üblichen Polyetherpolyolen aufweisen. Dies gilt insbesondere für diejenigen erfindungsgemäßen Verbindungen, die auf Polyetherpolyolen der Formel (IV) der oben beispielhaft genannten Art basieren.

Bei der erfindungsgemäßen Verwendung werden die erfindungsgemäßen Verbindungen im allgemeinen in Mengen von 1 bis 20 Gew.-%, bezogen auf das gesamte, zu Polyurethanschaumstoff ausreagierende Reaktionsgemisch eingesetzt. Im allgemeinen handelt es sich hierbei bei den erfindungsgemäßen Verbindungen lediglich um Hilfsmittel, die neben den üblichen Polyhydroxylverbindungen insbesondere Polyetherpolyolen und gegebenenfalls neben mitverwendetem Wasser als Reaktionspartner für die organischen Polyisocyanate zum Einsatz gelangen. Im Extremfall, insbesondere bei der Verwendung von erfindungsgemäßen Verbindungen auf Basis von realativ hochmolekularen Alkoholen der Formel (IV) können die erfindungsgemäßen Verbindungen jedoch auch, neben gegebenenfalls mitverwendetem Wasser, die einzige Reaktivkomponente für die organischen Polyisocyanate darstellen. Im allgemeinen werden die erfindungsgemäßen Verbindungen bei der erfindungsgemäßen Verwendung in solchen Mengen eingesetzt, daß in dem Reaktionsgemisch 0,01 bis 1 Gew.-% an tertiären Stickstoffatomen der erfindungsgemäßen Verbindungen vorliegen.

Wie bereits oben angedeutet, werden bei der erfindungsgemäßen Verwendung der erfindungsgemäßen Verbindungen vorzugsweise solche eingesetzt, die praktisch keine oder mindestens zwei Hydroxylgruppen pro Molekül aufweisen.

Neben der erfindungsgemäßen Verwendung können die erfindungsgemäßen Verbindungen auch noch für andere Einsatzgebiete Verwendung finden. Beispielhaft genannt seien die Verwendung als Oberflächen-aktive Mittel (auch in Form ihrer Ammoniumsalze) Emulgatoren oder als Stabilisatoren.

In den nachfolgenden Beispielen beziehen sich alle Prozentangaben auf das Gewicht.

### Beispiel 1

### 1a) Acetoacetylierung

1000 g (9,80 OH-Äquivalente) Polyethertriol der OH-Zahl 102, hergestellt aus Trimethylolpropan und Propylenoxid, 610 g (3,68 Mol) tert.-Butylacetoacetat werden bei 250 mbar auf ca. 105°C erhitzt, wobei die Destillation von tert.-Butanol einsetzt. Entsprechend der Destillationsgeschwindigkeit wird die Sumpftemperatur allmählich bis auf 130°C erhöht. Wenn die Destillation bei 250 mbar/130°C zum Erliegen kommt, wird der Druck schrittweise bis auf 20 mbar erniedrigt. Anschließend wird noch 2 Stunden an der Ölpumpe bei 0,5 mbar nachgerührt. Das resultierende Produkt hat eine durch Titration mit Tetrabutylammoniumhydroxid bestimmte Carbonyl-Zahl (CO-Zahl) von 153. Die Carbonylzahl (CO-Zahl) gibt die Menge an Keto-CO-Gruppen in mg pro g Substanz an, wobei im vorliegenden Fall sowohl die Keto- als auch die Enolform bei der Titration erfaßt wird. Daraus errechnet sich ein Umsatz von 36 % der anfangs vorhandenen Hydroxylgruppen.

### 1b) Aminierung

Zu 500 g (1,36 CO-Äquivalente) Zwischenprodukt gemäß Beispiel 1a) tropft man bei 25°C 135 g (1,33 Mol) 1-(Dimethylamino)-3-aminopropan innerhalb von 30 Minuten zu, wobei durch leichte Wasserkühlung eine Innentemperatur von 40 bis 50°C eingehalten wird. Nach Abklingen der exothermen Reaktion läßt man auf ca. 30°C abkühlen und legt unter Aufheizen auf 50°C allmählich Wasserpumpenvakuum ab, wobei die Destillation des Reaktionswassers einsetzt. Nach Destillation der Hauptmenge wird noch solange bei 80°C/20 mbar gerührt, bis der Wassergehalt <0,1 % liegt. Das klare gelbliche Produkt hat einen Gehalt an tert.-Aminstickstoff von 3,1 % und eine Viskosität von 3970 mPa.s/25°C.

### Beispiel 2

500 g (1,36 CO-Äquivalente) Zwischenprodukt gemäß Beispiel 1a) und 191,5 g (1,33 Mol) N-(3-Aminopropyl)-morpholin werden analog Beispiel 1b) eingesetzt. Das klare gelbliche Produkt hat einen Gehalt an tert.-Aminstickstoff von 2,8 % und eine Viskosität von 1120 mPa.s/25°C.

### Beispiel 3

### 3a) Acetoacetylierung

4900 g (21,88 OH-Äquivalente) Polyethertriol der OH-Zahl 250, hergestellt aus Glycerin und Propylenoxid, und 2419 g (15,29 Mol) tert.-Butylacetoacetat werden analog Beispiel 1a) umgesetzt. Das resultierende Produkt hat eine CO-Zahl von 117,6. Daraus erreichnet sich ein Umsatz von 57 % der anfangs vorhandenen Hydroxylgruppen.

### 3b) Aminierung

800 g (1,68 CO-Äquivalente) Zwischenprodukt gemäß Beispiel 3a) und 171 g (1,68 Mol) 1-(Dimethylamino)-3-aminopropan werden analog Beispiel 1b) umgesetzt. Das klare gelbliche Produkt hat einen Gehalt an tert.-Aminstickstoff von 2,5 % und eine Viskosität von 1300 mPa.s/25°C.

### Beispiel 4

### 4a) Acetoacetylierung

4000 g (17,86 OH-Äquivalente) Polyethertriol der OH-Zahl 250, hergestellt durch Propoxylierung von Trimethylolpropan und anschließende Ethoxylierung des Propoxylierungsproduktes (PO:EO-Gewichtsverhältnis = 1,1:98,9), und 3668 g (23,2 Mol) tert.-Butylacetoacetat werden analog Beispiel 1a) umgesetzt. Das resultierende von überschüssigem t-Butylacetat destillativ befreite Produkt hat eine CO-Zahl von 178,5. Daraus errechnet sich ein Umsatz von 97,3 % der anfangs vorhandenen Hydroxylgruppen.

### 4b) Aminierung

471 g (1,5 CO-Äquivalente) Zwischenprodukt gemäß Beispiel 4a) und 152 g (1,5 Mol) 1-(Dimethylamino)-3-aminopropan werden analog Beispiel 1b) umgesetzt. Das klare gelbliche Produkt hat einen Gehalt an tert.-Aminstickstoff von 3,5 % und eine Viskosität von 1070 mPa.s/25°C.

### Beispiel 5

### 5a) Acetoacetylierung

1000 g (3,3 Äquivalente) Dimethylpolysiloxandiol der OH-Zahl 198 und 669 g (4,24 Mol) tert.-Butylacetoacetat werden analog Beispiel 1a) umgesetzt. Das resultierende von überschüssigem t-Butylacetat destillativ befreite Produkt hat eine CO-Zahl von 140,5. Daraus errechnet sich ein Umsatz von 80,8 % der anfangs vorhandenen Hydroxylgruppen.

### 5b) Aminierung

800 g (2,0 CO-Äquivalente) Zwischenprodukt gemäß Beispiel 5a) und 204 g (2,0 Mol) 1-(Dimethylamino)-3-aminopropan werden analog Beispiel 1b) umgesetzt. Das klare gelbliche Produkt hat einen Gehalt an tert.-Aminstickstoff von 2,9 % und eine Viskosität von 50 mPa.s/25°C.

### Beispiel 6

### 6a) Acetoacetylierung

1007 g (8,06 OH-Äquivalente) Polyetherhexanol der OH-Zahl 450, hergestellt aus Sorbit und Propylenoxid und 1654 g (10,47 Mol) tert.-Butylacetoacetat werden analog Beispiel 1a) umgesetzt und von überschüssigem t-Butylacetat destillativ befreit. Daraus errechnet sich ein Umsatz von 92 % der anfangs vorhandenen Hydroxylgruppen.

### 6b) Aminierung

500 g (2,28 CO-Äquivalente) Zwischenprodukt gemäß Beispiel 6a) und 116 g (1,14 Mol) 1-(Dimethylamino)-3-aminopropan werden analog Beispiel 1b) umgesetzt. Das klare hellbraune Produkt hat einen Gehalt an tert.-Aminstickstoff von 2,7 % und eine Viskosität von 13060 mPa.s/25°C.

### Beispiel 7

### 7a) Acetoacetylierung

5000 g (5,0 OH-Äquivalente) Polyetherdiol der OH-Zahl 56, hergestellt aus Propylenglykol und Propylenoxid, und 1028 g (6,5 Mol) tert.-Butylacetoacetat werden analog Beispiel 1a) umgesetzt. Das resultierende von überschüssigem Butylacetat destillativ befreite Produkt hat eine CO-Zahl von 51. Daraus errechnet sich ein Umsatz von 98,4 % der anfangs vorhandenen Hydroxylgruppen.

### 7b) Aminierung

600 g (0,55 CO-Äquivalente) Zwischenprodukt gemäß Beispiel 7a) und 77 g (0,54 Mol) N-(3-Aminopropyl)-piperidin werden analog Beispiel 1b) umgesetzt. Das klare gelbliche Produkt hat einen Gehalt an Aminstickstoff von 1,1 % und eine Viskosität von 1010 mPa.s/25°C.

### Verwendungsbeispiele

### Polyolformulierung

80 Gew.-Teile eines Polyetherpolyols der OH-Zahl 28, hergestellt durch Propoxylierung von Trimethylolpropan und anschließende Ethoxylierung des Propoxylierungsprodukts (PO:EO-Gewichtsverhältnis = 82,5:17,5), sowie 20 Gew.-Teile eines analog aufgebauten, mit 20 % Styrol/Acrylnitril (4:6) gepfropften Polyetherpolyols der OH-Zahl 28 werden in 5 Parallelversuchen mit der in Tabelle 1 angegebenen Menge an Aktivator vermischt. Als Treibmittel werden jeweils 2,0 Gew.-Teile Wasser zugegeben.

### Polyisocyanatkomponente

In den nachfolgenden Beispielen wurde jeweils ein Polyisocyanatgemisch der Diphenylmethanreihe (Gemisch aus Diisocyanatodiphenylmethan-Isomeren und deren höheren Homologen) der Viskosität (23°C) 200 mPa.s mit einem NCO-Gehalt von 32 Gew.-% verwendet.

### Herstellung von Schaumstoffen

Die Herstellung der Schaumstoffe erfolgte jeweils nach der Methode der Handverschäumung. Dabei werden alle Komponenten mit Ausnahme der Polyisocyanatkomponente während 30 s vorgerührt (Rührgeschwindigkeit: 1000 Umdrehungen/Min.). Anschließend wird die Polyisocyanatkomponente zugesetzt und während weiterer 10 s bei Raumtemperatur weiter gerührt. Das Mischungsverhältnis beträgt in allen Beispielen 100:42, entsprechend einer Isocyanatkennzahl von 120.

Die Reaktivität der Polyolkomponente wurde durch die Start-, Steig- und Abbindezeiten in Parallelversuchen bestimmt, wobei die Polyolformulierung wie beschrieben mit der Polyisocyanatkomponente in einem Becherglas unter Rühren bei Raumtemperatur vereinigt wird. Die Startzeit ist die Zeit, die vom Zeitpunkt der Polyisocyanatzugabe bis Beginn des Schäumvorgangs verstreicht; die Steigzeit ist die Zeit, die vom Zeitpunkt der Polyisocyanatzugabe bis zur Beendigung des Schäumvorgangs verstreicht; die Abbindezeit ist die Zeit, die vom Zeitpunkt der Polyisocyanatzugabe bis zur Klebfreiheit des Schaumstoffs verstreicht.

**Tabelle 1**

| verwendeter Aktivator | aus Beispiel 1 | aus Beispiel 3 | aus Beispiel 4 | Vergleich 1 | Vergleich 2 |
|---|---|---|---|---|---|
| Aktivatormenge (Gew.-Teile) | 4,0 | 10,0 | 4,0 | 11,0 | 10,0 |
| Startzeit (s) | 32 | 18 | 24 | 38 | n.b.∗ (>60) |
| Steigzeit (s) | 180 | 121 | 120 | 205 | n.b. (>600) |
| Abbindezeit (s) | 195 | 130 | 145 | 240 | n.b.∗ (>600) |

| | | | | | |
|---|---|---|---|---|---|
| ∗n.b. = nicht bestimmt (die Reaktionszeiten glichen der Reaktionszeit eines nicht aktivierten Reaktionsgemischs) | | | | | |

Wie der Tabelle 1 zu entnehmen wurden erfindungsgemäße Aktivatoren gemäß Beispielen 1, 3 und 4 mit tert.-Aminogruppen aufweisenden Polyetherpolyolen gemäß Stand der Technik verglichen. Bei dem Aktivator gemäß Vergleich 1 handelt es sich um ein tert.-Aminogruppen aufweisendes Polyetherpolyol der OH-Zahl 500, hergestellt durch Propoxylierung von Triethanolamin. Bei dem Aktivator gemäß Vergleich 2 handelt es sich um ein tert.-Aminogruppen aufweisendes Polyetherpolyol der OH-Zahl 60, hergestellt durch Propoxylierung von Ethylendiamin.

## Patentansprüche

1. Tertiäre Aminogruppen aufweisende Verbindungen der Formel (I) 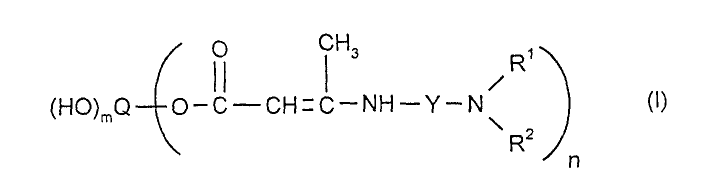 in welcher
Q für den Rest steht, der durch Entfernung der Hydroxylgruppe(n) aus einem von 1,4-Butandiol verschiedenen 2- bis 6-wertigen Alkohol des Molekulargewichts 62 bis 4000 erhalten wird,
Y für einen zweiwertigen aliphatischen Kohlenwasserstoffrest mit 2 bis 6 Kohlenstoffatomen steht, wobei zwischen den beiden Stickstoffatomen mindestens zwei Kohlenstoffatome angeordnet sind,
R¹ und R² für gleiche oder verschiedene Reste stehen und Alkylreste bedeuten, die zusammen mit dem Stickstoffatom und gegebenenfalls weiteren Heteroatomen auch zu einem gesättigten heterocyclischen Ring verknüpft sein können,
m eine Zahl von 0 bis 5 und
n eine Zahl von 1 bis 6 bedeuten, mit der Maßgabe, daß die Summe m + n eine Zahl von 2 bis 6 ergibt.

2. Tertiäre Aminogruppen aufweisende Verbindungen der Formel (I), für welche
Q für den Rest steht, wie er durch Entfernung der Hydroxylgruppen aus einem 2- bis 6-wertigen Alkohol des Molekulargewichtsbereichs 106 bis 4000 erhalten wird,
Y für einen Ethylen-, 1,2-Propylen- oder Trimethylenrest steht,
R¹ und R² für gleiche C₁-C₃-Alkylreste stehen oder zusammen mit dem Stickstoffatom und gegebenenfalls einem Sauerstoffatom zu einem gesättigten heterocyclischen Ring mit 6 Ringgliedem verknüpft sind,
m für eine Zahl von 0 bis 5 und
n für eine Zahl von 1 bis 6 stehen, mit der Maßgabe. daß die Summe m + n eine Zahl von 2 bis 6 ergibt.

3. Tertiäre Aminogruppen aufweisende Verbindungen der Formel (I) gemäß Anspruch 1 oder 2, **dadurch gekennzeichnet, daß**
Q für den Rest steht, wie er durch Entfernung der Hydroxylgruppen aus einem di- bis hexafunktionellen Polyetherpolyols des Molekulargewichtsbereichs 200 bis 2000 erhalten wird.

4. Verfahren zur Herstellung von tert.-Aminogruppen aufweisenden Verbindungen gemäß Anspruch 1, **dadurch gekennzeichnet, daß** man Verbindungen der Formel (II) 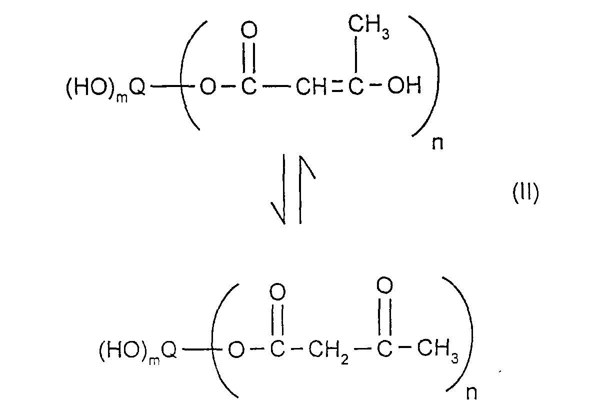 mit Verbindungen der Formel (III) 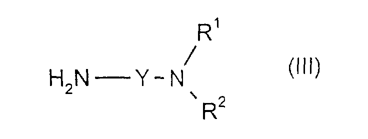 im Sinne einer Kondensationsreaktion zu Reaktionen bringt, wobei
Q, Y, R¹, R², m und n die in Anspruch 1 genannte Bedeutung haben.

5. Verfahren gemäß Anspruch 4, **dadurch gekennzeichnet, daß** Verbindungen der Formeln (II) und (III) eingesetzt werden, für welche
Q, Y, R¹, R², m und n die in Anspruch 2 oder Anspruch 3 genannte Bedeutung haben.

6. Verwendung der tert.-Aminogruppen aufweisenden Verbindungen der Formel (1) 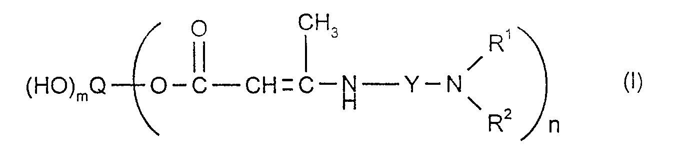 in welcher
Q für den Rest steht, der durch Entfernung der Hydroxylgruppe(n) aus einem von 1,4-Butandiol verschiedenen 2- bis 6-wertigen Alkohol des Molekulargewichts 62 bis 4000 erhalten wird,
Y für einen zweiwertigen aliphatischen Kohlenwasserstoffrest mit 2 bis 6 Kohlenstoffatomen steht, wobei zwischen den beiden Stickstoffatomen mindestens zwei Kohlenstoffatome angeordnet sind.
R¹ und R² für gleiche oder verschiedene Reste stehen und Alkylreste bedeuten, die zusammen mit dem Stickstoffatom und gegebenenfalls weiteren Heteroatomen auch zu einem gesättigten heterocyclischen Ring verknüpft sein können,
m eine Zahl von 0 bis 5 und
n eine Zahl von 1 bis 6 bedeuten, mit der Maßgabe, daß die Summe m + n eine Zahl von 2 bis 6 ergibt,
als gegebenenfalls einbaufähige Katalysatoren für die Isocyanat-Additionsreaktion bei der Herstellung von Polyurethanschaumstoffen nach dem Isocyanat-Polyadditionsverfahren.

7. Verwendung gemäß Anspruch 6 als Katalysatoren bei der Herstellung von Verbundkörpern aus Kunststoffolien und Polyurethanschaumstoff durch Hinterschäumen von Kunststoffolien mit einem zu Polyurethanschaumstoff ausreagierenden Reaktionsgemisch.

## Claims

1. Compounds of formula (I) which contain tertiary amino groups 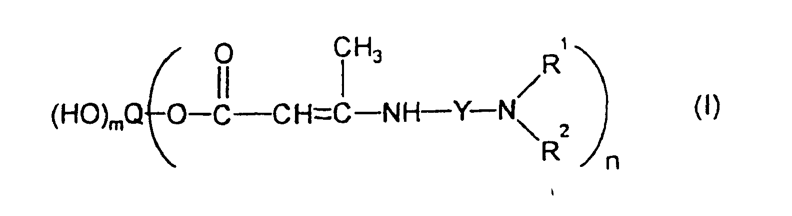 wherein
Q represents the radical obtained by removal of the hydroxyl group(s) from a di- to hexahydric alcohol which is different from 1,4-butanediol and which has a molecular weight ranging from 62 to 4000,
Y represents a divalent aliphatic hydrocarbon radical containing 2 to 6 carbon atoms, wherein at least two carbon atoms are disposed between the two nitrogen atoms,
R¹ and R² represent identical or different radicals and denote alkyl radicals which can also be joined, together with the nitrogen atom and optionally together with other hetero atoms, to form a saturated heterocyclic ring,
m denotes a number from 0 to 5, and
n denotes a number from 1 to 6, with the proviso that the sum m + n is a number from 2 to 6.

2. Compounds of formula (I) which contain tertiary amino groups, wherein
Q represents the radical obtained by removal of the hydroxyl groups from a di- to hexahydric alcohol with a molecular weight ranging from 106 to 4000,
Y represents an ethylene, 1,2-propylene or trimethylene radical,
R¹ and R² represent identical or different C₁-C₃ alkyl radicals or are joined, together with the nitrogen atom and optionally together with an oxygen atom, to form a saturated, 6-membered heterocyclic ring,
m represents a number from 0 to 5 and
n represents a number from 1 to 6, with the proviso that the sum m + n is a number from 2 to 6.

3. Compounds of formula (I) which contain tertiary amino groups according to claims 1 or 2, **characterised in that**
Q represents the radical obtained by removal of the hydroxyl groups from a di- to hexafunctional polyether polyol with a molecular weight ranging from 200 to 2000.

4. A process for the production of the compounds containing tertiary amino groups according to claim 1, **characterised in that** compounds of formula (II) 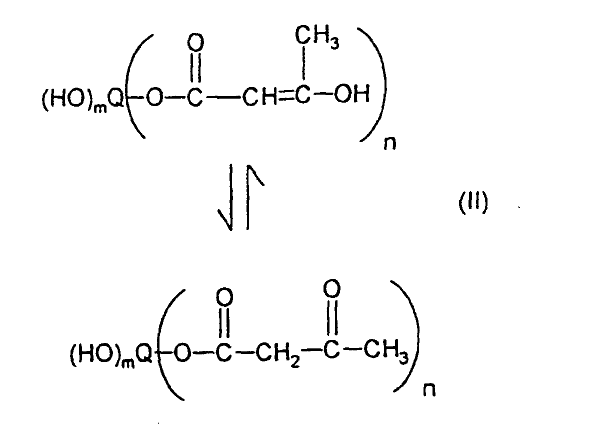 are reacted, in the sense of a condensation reaction, with compounds of formula (III) 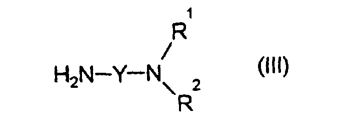 where Q, Y, R¹, R², m and n have the meanings given in claim 1.

5. A process according to claim 4, **characterised in that** compounds of formulae (II) and (III) are used in which Q, Y, R¹, R², m and n have the meanings given in claims 2 or 3.

6. Use of the compounds of formula (I) containing tertiary amino groups 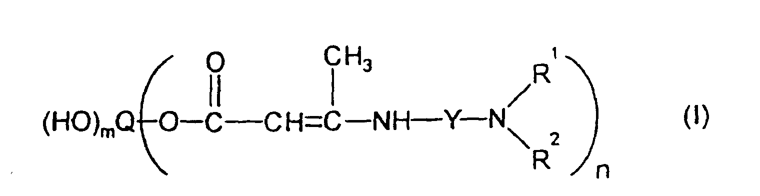 wherein
Q represents the radical obtained by removal of the hydroxyl group(s) from a di- to hexahydric alcohol which is different from 1,4-butanediol and which has a molecular weight ranging from 62 to 4000,
Y represents a divalent aliphatic hydrocarbon radical containing 2 to 6 carbon atoms, wherein at least two carbon atoms are disposed between the two nitrogen atoms,
R¹ and R² represent identical or different radicals and denote alkyl radicals which can also be joined, together with the nitrogen atom and optionally together with other hetero atoms, to form a saturated heterocyclic ring,
m denotes a number from 0 to 5, and
n denotes a number from 1 to 6, with the proviso that the sum m + n is a number from 2 to 6,
as catalysts for an isocyanate addition reaction, which catalysts can optionally be incorporated in the production of polyurethane foams by an isocyanate polyaddition process.

7. A use according to claim 6 as catalysts in the production of composite bodies from plastics sheeting and polyurethane foam by back-foaming of plastics sheeting with a reaction mixture which reacts out to form polyurethane foam.

## Revendications

1. Composés présentant des groupes amino tertiaires répondant à la formule (I) 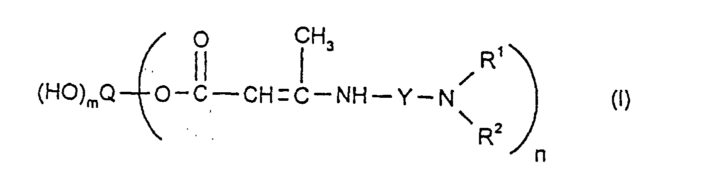 dans laquelle
Q représente le radical que l'on obtient par élimination du ou des groupes hydroxyle d'un alcool divalent à hexavalent différent du 1,4-butanediol, possédant un poids moléculaire de 62 à 4000,
Y représente un radical d'hydrocarbure aliphatique divalent contenant de 2 à 6 atomes de carbone, au moins deux atomes de carbone étant disposés entre les deux atomes d'azote,
R¹ et R² représentent des radicaux identiques ou différents et désignent des radicaux alkyle qui peuvent également être liés, ensemble avec l'atome d'azote et le cas échéant avec d'autres hétéroatomes, pour former un noyau hétérocyclique saturé,
m représente un nombre de 0 à 5, et
n représente un nombre de 1 à 6, avec cette mesure que la somme m + n est égale à un nombre de 2 à 6.

2. Composés présentant des groupes amino tertiaires répondant à la formule (I), pour lesquels
Q représente le radical que l'on obtient par élimination du ou des groupes hydroxyle d'un alcool divalent à hexavalent du domaine de poids moléculaire de 106 à 4000,
Y représente un radical d'éthylène, de 1,2-propylène ou de triméthylène,
R¹ et R² représentent des radicaux alkyle en C₁-C₃ identiques ou sont liés, ensemble avec l'atome d'azote et le cas échéant avec un atome d'oxygène, pour former un noyau hétérocyclique saturé contenant 6 termes cycliques,
m représente un nombre de 0 à 5, et
n représente un nombre de 1 à 6, avec cette mesure que la somme m + n est égale à un nombre de 2 à 6.

3. Composés présentant des groupes amino tertiaires répondant à la formule (I) selon la revendication 1 ou 2, **caractérisés en ce que**
Q représente le radical que l'on obtient par élimination des groupes hydroxyle d'un polyétherpolyol di- à hexafonctionnel, du domaine de poids moléculaire de 200 à 2000.

4. Procédé pour la préparation de composés présentant des groupes amino tertiaires selon la revendication 1, **caractérisé en ce qu'**on amène à réagir des composés répondant à la formule (II) 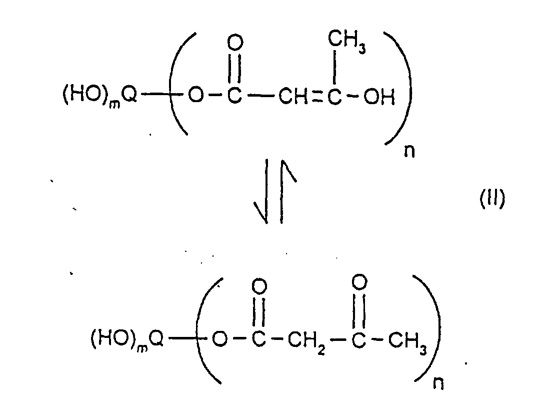 avec des composés répondant à la formule (III) 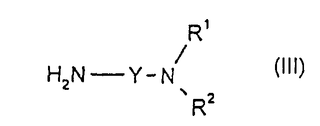 dans le sens d'une réaction de condensation, dans lequel
Q, Y, R¹, R², m et n ont la signification indiquée à la revendication 1.

5. Procédé selon la revendication 4, **caractérisé en ce qu'**on met en oeuvre des composés répondant aux formules (II) et (III), pour lesquels
Q, Y, R¹, R², m et n ont la signification indiquée à la revendication 2 ou à la revendication 3.

6. Utilisation des composés présentant des groupes amino tertiaires répondant à la formule (I) 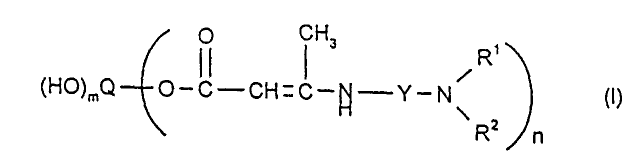 dans laquelle
Q représente le radical que l'on obtient par élimination du ou des groupes hydroxyle d'un alcool divalent à hexavalent différent du 1,4-butanediol, possédant un poids moléculaire de 62 à 4000,
Y représente un radical d'hydrocarbure aliphatique divalent contenant de 2 à 6 atomes de carbone, au moins deux atomes de carbone étant disposés entre les deux atomes d'azote,
R¹ et R² représentent des radicaux identiques ou différents et désignent des radicaux alkyle qui peuvent également être liés, ensemble avec l'atome d'azote et le cas échéant avec d'autres hétéroatomes, pour former un noyau hétérocyclique saturé,
m représente un nombre de 0 à 5, et
n représente un nombre de 1 à 6, avec cette mesure que la somme m + n est égale à un nombre de 2 à 6.
à titre de catalyseurs le cas échéant aptes à être incorporés pour la réaction d'addition d'isocyanates lors de la préparation de mousses de polyuréthanne conformément au procédé de polyaddition d'isocyanates.

7. Utilisation selon la revendication 6, à titre de catalyseurs lors de la préparation de corps composites constitués par des feuilles en matière synthétique et par de la mousse de polyuréthanne, par application sur la face dorsale de feuilles en matière synthétique d'un mélange réactionnel qui, au terme de sa mise en réaction, donne lieu à une mousse de polyuréthanne.
